# EUROPEAN PATENT APPLICATION

(11) **EP 2 022 392 A2**
(43) Date of publication of application: **11.02.2009**
(21) Application number: 08160969.5
(22) Date of filing: 23.07.2008
(51) Int. Cl.: A61B 3/16

(54) **Non-contact type tonometer**

(30) Priority: 27.07.2007 JP 2007195340
(71) Applicant: Kabushiki Kaisha TOPCON, Tokyo 174-8580 (JP)
(72) Inventor: Naito, Tomoko c/o Kabushiki Kaisha TOPCON, Tokyo 174-8580 (JP); Yanagi, Eiichi c/o Kabushiki Kaisha TOPCON, Tokyo 174-8580 (JP); Sagehashi, Hideo c/o Kabushiki Kaisha TOPCON, Tokyo 174-8580 (JP)
(74) Representative: Ilgart, Jean-Christophe

(57) **Abstract**

A non-contact type tonometer includes a air injector (1) for blowing air to a cornea (C) of a subject's eye (E) along a reference axis to deform the cornea, a projection optical system (5) for projecting projection light onto the subject's eye, a light-receiving optical system (6) for receiving reflected light which is projected from the projection optical system and reflected on the subject's eye, an alignment unit for positioning the projection optical system and the light-receiving optical system in the reference axis direction based on an amount of the reflected light which is received by the light-receiving optical system and an intraocular pressure detector for detecting a deformation of the cornea based on the reflected light amount. The projection optical system includes a light flux shape regulator (52) which regulates the projection light such that the projection light has a non-circular shape extending in a predetermined direction.

## Description

### BACKGROUND OF THE INVENTION

### Field of the Invention

The present invention relates to a non-contact type tonometer which blows air on a cornea of a subject's eye and detects a state of a deformation of the cornea of the subject's eye to measure an intraocular pressure value of the subject's eye.

### Description of the Related Art

Heretofore, various non-contact type tonometers (also referred to as "non-contact tonometer") have been known. Such a tonometer blows air onto a cornea of a subj ect' s eye with a nozzle to applanate the cornea, and measures an intraocular pressure value of the subject's eye on the basis of an internal pressure of a chamber when the cornea is deformed into a certain shape.

Of such non-contact type tonometers, one known tonometer includes five light sources, which are independently set, such as an anterior segment lighting light source, a fixation target light source, an XY alignment light source, a Z alignment light source, and an applanation detection light source (e.g., refer to Japanese Patent Application Publication No. 2002-165763).

Further, another known non-contact type tonometer uses one common light source as the XY alignment light source and the applanation detection light source. In this tonometer, four light sources such as the anterior segment lighting light source, the fixation target light source, an XY alignment/applanation detection light source, and the Z alignment light source are independently set (e.g., refer to Japanese Patent Application Publication No. 2006-334435).

Here, the "XY alignment" represents a positional relationship between an instrument and the cornea of the subject's eye in the two-dimensional coordinate system with axes X and Y. That is, in the XY alignment, the instrument is relatively adjusted in left, right, upward, and downward directions in relation to the cornea of the subject's eye. The positional relationship is detected as a movement of a luminescent spot on a two-dimensional sensor in a way that the movement is obtained by projecting light on a subject's s eye from a front direction and receiving a reflected light from the cornea of the subject's s eye.

Further, the "Z alignment" represents a positional relationship between the instrument and the subject's cornea in forward and backward directions in relation to the cornea of the subject's eye in a one-dimensional coordinate system with axis Z. The positional relationship is detected as a movement of a luminescent spot on a one-dimensional sensor in a way that the movement is obtained by obliquely projecting light on a subject's eye and receiving a reflected light from the cornea of the subject's eye.

Incidentally, an "alignment adjustment" represents an adj ustment in which the positional relationship between the instrument and the subject's cornea are adjusted in the left, right, upward and downward directions and in the forward and backward directions to align the instrument in a predetermined position based on the detections of the "XY alignment" and the "Z alignment". Immediately after the alignment adjustment is completed, the intraocular pressure value of the subject' s eye is measured by blowing air on the cornea of a subject's eye from the nozzle.

However, since the non-contact type tonometer described in Japanese Patent Application Publication No. 2002-165763 uses the five light sources, which are independently set, the tonometer requires a complex optical system due to the installation of an independent projection system for each light source. This causes a problem in that the instrument becomes large.

In the case of the non-contact type tonometer described in Japanese Patent Application Publication No. 2006-334435, the cornea of a subjects eye is illuminated by a circular light flux from the common light source for the XY alignment and the applanation detection. Since a range illuminated by the circular light flux is used for the XY alignment and the applanation detection, this tonometer has a problem in that the tonometer fails to secure both an intraocular pressure value measurement accuracy in the applanation and a range needed for the XY alignment.

More specifically, when the range illuminated by the circular light beam is increased, the range needed for the XY alignment can be increased, but the intraocular pressure value measurement accuracy in the applanation is deteriorated. In contrast, when the range illuminated by the circular light beam is reduced, the intraocular pressure value measurement accuracy in the applanation is secured, but the range needed for the XY alignment is reduced. In particular, although there is a demand for increasing a range needed for an alignment as large as possible for an auto-alignment tonometer performing an alignment automatically, these conventional tonometers fail to meet such a demand.

### SUMMARY OF THE INVENTION

The present invention has been made in light of the above-described problems, and an object of the present invention is to provide a non-contact type tonometer which enables simultaneous securement of an intraocular pressure value measurement accuracy with applanation and of the range needed for a Z alignment, while making an optical system compact and relatively simple.

To achieve the above object, a nor-contact type tonometer according to an embodiment of the present invention, which deforms a cornea of a subject's eye in a non-contact state and measures an intraocular pressure value, includes a compressed air injector configured to blow air to the cornea of the subj ect' s eye along a reference axis to deform the cornea of the subj ect' s eye, a projection optical system configured to project projection light onto the subject's eye, a light receiving optical system configured to receive reflected light which is projected from the projection optical system and reflected on the subject's eye, an alignment unit configured to position the projection optical system and the light receiving optical system by moving the projection optical system and the light receiving optical system relative to the subj ect' s eye in the direction of the reference axis, based on a light amount of the reflected light, which is received by the light receiving optical system, and an intraocular pressure detector configured to detect a deformation of the cornea of the subject's eye deformed by the compressed air injector, based on the reflected light amount received by the light receiving optical system. The projection optical system includes a light flux shape regulator which regulates the projection light such that the projection light has a non-circular shape extending in a predetermined direction.

### BRIEF DESCRIPTION OF THE DRAWINGS

FIG.1 is a plan arrangement view showing an optical system of a non-contact type tonometer of a first embodiment.
FIG.2 is a side arrangement view showing the optical system of the non-contact type tonometer of the first embodiment.
FIG.3 is an enlarged front view showing an aperture of a Z alignment/applanation detection projection system of the non-contact type tonometer of the first embodiment.
FIG.4 is a view showing characteristics of light amount changes due to applanation during an intraocular pressure measurement and a characteristic of internal pressure change in a chamber, in a case of the non-contact type tonometer of the first embodiment.
FIG.5 is a plan view showing a necessary range of illumination for a Z alignment at a time when the cornea of a subject's eye moves in a Z direction, and an optimum range of illumination for the applanation.
FIG.6 is an explanatory view showing a lateral movement of a luminescent spot made by a light source in the cases where a distance between the cornea of a subj ect' s eye and a nozzle is optimum, small, and large.
FIG.7 is a view comparing the characteristics of Comparative Example 1, Comparative Example 2, and the first embodiment when a light amount of a Z alignment light receiving sensor changes due to a movement of the cornea of a subject's eye.
FIG. 8 is a front view showing Variation of the shape of an aperture.
FIG. 9 is a front view showing Variation 2 of the shape of an aperture.

### DETAILED DESCRIPTION OF THE PREFERRED EMBODIMENTS

A preferred embodiment for carrying out a non-contact type tonometer of the present invention is described below with reference to a first embodiment shown in the accompanying drawings.

First, a configuration is described.

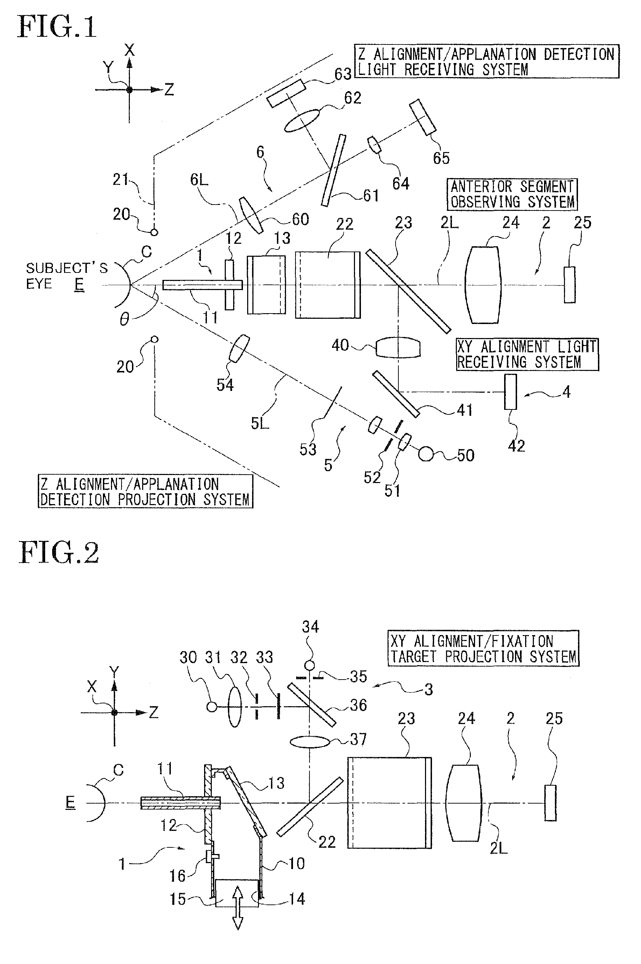

FIG.1 is a plan arrangement view showing an optical system of a non-contact type tonometer of the first embodiment. FIG. 2 is a side arrangement view showing the optical system thereof.

The non-contact type tonometer of the first embodiment is configured to measure an intraocular pressure by deforming the cornea of a subject's eye E in a non-contact state. As shown in FIG.1, this non-contact type tonometer includes: a compressed air injection structure 1, serving as a compressed air injection portion, which blows air on the cornea of the subject's eye E along a reference axis; a Z alignment/applanation detection projection system 5, serving as a projection optical system, which causes projection light incident on the subject's eye E; a Z alignment/applanation detection light receiving system 6, serving as a light receiving optical system, which receives a reflection light projected from the Z alignment/applanation detection projection system 5 and reflected by the subject's eye E; an alignment portion (not shown) which moves the Z alignment/applanation detection projection system 5 and the Z alignment/applanation detection light receiving system 6 in a direction of the reference axis to position them relative to the subject's eye E, based on an amount of the reflection light received by the Z alignment/applanation detection light receiving system 6; and an intraocular pressure detector (not shown) which detects a deformation of the cornea of the subject's eye E, cased by the air injected from the compressed air injection structure 1, using an amount of the reflection light received by the Z alignment/applanation detection light receiving system 6.

The Z alignment/applanation detection projection system 5 includes a light flux shape regulator, which regulates a projection light into a non-circular shape extending in a predetermined direction. When the Z alignment/applanation detection projection system 5 and the Z alignment/applanation detection light receiving system 6 are moved in the direction of the reference axis, the projection light moves on the subject's eye E in a direction in which the projection light having the non-circular shape is extended. Here, the reference axis is the forward and backward direction of, for example, the cornea of a subject's eye relative to an instrument.

As shown in FIGs. 1 and 2, the compressed air injection structure 1 blows the air in the Z direction, being the direction of the reference axis, so as to deform, for example, flatten, the cornea C of a subject's eye. The non-contact type tonometer of this embodiment, further, includes an anterior segment observing system 2, an XY alignment/fixation target projection system 3, and an XY alignment light receiving system 4. The alignment portion is configured to move the Z alignment/applanation detection projection system 5 and the Z alignment/applanation detection light receiving system 6 in an X and Y directions, for example, in FIG. 1, relative to the subject's eye E, based on an amount of the reflection light received by the XY alignment light receiving system 4. The direction in which the circular projection light extends is preferably a direction along a plane including the reference axis and an optical axis of the projection light in the case where the projection light from the Z alignment/applanation detection projection system 5 has an angle with the reference axis. This direction is described as an X direction in FIGs. 1 and 2, and hereinafter also referred to as a "lateral direction".

Each constituent element will be described below.

### [Compressed Air Injection Structure]

As show in FIGs.1 and 2, the compressed air injection structure 1 includes a chamber 10, a nozzle 11, an anterior segment window glass 12, a chamber window glass 13, a cylinder 14, a piston 15, and a chamber internal pressure sensor 16.

That is, the air compressed in the cylinder 14 with the piston 15, which is driven by a not illustrated solenoid, is blown on the cornea C of the subject's eye, via the chamber 10 and the nozzle 11. Incidentally, in the chamber 10, the chamber internal pressure sensor 16 detecting the pressure change in the chamber 10 is set.

### [Anterior Segment Observing System]

As shown in FIGs.1 and 2, in the anterior segment observing system 2, a plurality of anterior segment illumination light sources 20, 20 directly illuminating an anterior segment of the subject's eye E with an observing light, are provided in an instrument case 21. Moreover, on an anterior segment observing optical axis 2L (=nozzle axis, that is, the reference axis), included are the nozzle 11, the anterior segment window glass 12, the chamber window glass 13, a first half mirror 22, a second half mirror 23, an objective lens 24, andaCCD25 (Charge Coupled Devices), in this order from the cornea C of the subject' s eye.

An anterior segment image of the subject's eye E illuminated with the anterior segment illumination light sources 20, 20 passes inside and outside the nozzle 11, and also passes through the anterior segment window glass 12, the chamber window glass 13, the first half mirror 22, and the second half mirror 23. Accordingly, the image is focused with the objective lens 24 and imaged on the CCD 25 which is a charge-coupled device. This CCD 25 is an image sensor connected to a not illustrated monitor, and displays the anterior segment image of the subject's eye E on a monitor screen set on a position where an observer can observe. Incidentally, a luminescent spot image being an XY alignment target light is also displayed on the monitor screen in addition to the anterior segment image.

### [XY Alignment/Fixation Target Projection System]

As shown in FIG.2, the XY alignment/fixation target projection system 3 includes a light source 30 for XY alignment emitting infrared light, a condensing lens 31, an aperture diaphragm 32, a pin hole plate 33, a light source 34 emitting visible light for fixation target, a pin hole plate 35, a dichroic mirror 36 which is disposed on a light path such that a focal point coincides with the pin hole plates 33, 35, and a collimator lens 37. Incidentally, the "dichroic mirror" is a type of mirror formed using special optical material, and it reflects light having a certain wavelength and transmits light having other wavelength.

Infrared light emitted from the light source 30 for XY alignment is focused with the condensing lens 31. The infrared light thus focused passes through the aperture diaphragm 32, and is led to the pin hole plate 33. A light flux passed through the pin hole plate 33 is reflected by the dichroic mirror 36, and caused to become a parallel light flux by the collimator lens 37. Then the light flux is reflected by the first half mirror 22. Thereafter, the light flux passes through the chamber window glass 13 and then the inside the nozzle 11 to form an XY alignment target light. Further, the XY alignment target light is reflected by a surface of the cornea such that a luminescent spot image is formed on an intermediate position between a vertex of the cornea C of the subject's eye and a center of curvature of the cornea C thereof. Incidentally, the aperture diaphragm 32 is disposed on a position that is conjugate to the vertex of the cornea C with respect to the collimator lens 37.

Fixation target light emitted from the light source 34 for fixation target passes through the pin hole plate 35 and the dichroic mirror 36, and is caused to become parallel light by the collimator lens 37. The light is reflected by the first half mirror 22, thereafter, passes through the chamber window glass 13 and then the inside of the nozzle 11, and is led to the subject's eye E. when measuring an intraocular pressure, the subject gazes at this fixation target light as a fixation target such that a subject's visual line is fixed, whereby the motion of the subject's eye E is checked.

### [XY Alignment Light Receiving System]

The XY alignment light receiving system 4 is an optical system to detect an eccentricity between the meter case 21 and the cornea C of the subject's eye. As shown in FIG.1, the XY alignment light receiving system 4 includes an imaging lens 40, a reflecting mirror 41, and an XY alignment light receiving sensor 42. Incidentally, for the XY alignment light receiving sensor 42, a PSD sensor is used.

Here, the "PSD sensor" is a light sensor capable of detecting the position of a spot-like light using a PSD (Position Sensitive Detector: a semiconductor position detecting element). The sensor basically has a PIN structure with a single joint surface such as a photodiode, but it has large surface area of, for example, 1 mm by 12 mm or 10 mm by 10 mm. The applying of a spot of light on the semiconductor surface generates a charge. The generated charge reaches electrodes on both ends and an amount of the reached charge is inversely proportional to the distance from the position of the spot of light to the electrode. Therefore, by performing necessary calculation, current taken out from the electrode can be used as data proportional to the position of the spot of light. Incidentally, there are one-dimensional PSD sensors and two-dimensional PSD sensors depending on the structures of the semiconductor surface and the electrode. A PSD sensor responds very quickly and has high reliability in position recognition because the operations thereof are very simple as described above. Further, since resolution is extremely high, it has high position detection accuracy.

A flux of reflected light projected on the cornea C of the subject's eye by the XY alignment/fixation target projection system 3 and reflected by the surface of the cornea passes inside the nozzle 11 and transmits through the chamber window glass 13 and the first half mirror 22. The part of the flux is reflected by the second half mirror 23. The light flux reflected by the second half mirror 23 is formed into an image of luminescent spot by the imaging lens 40 and is reflected by the reflecting mirror 41, such that a luminescent spot image is formed on the XY alignment light receiving sensor 42.

### [Z Alignment/Applanation Detection Projection System]

As shown in FIG.1, the Z alignment/applanation detection projection system 5 includes: a common light source 50 emitting infrared light; a condensing lens 51 and a pin hole plate 53 disposed on a projection optical axis 5L being a line connecting the common light source 50 and the cornea C of the subject's eye; an aperture 52 disposed on the axis 5L and disposed on a position substantially conjugate to the subject's eye E between the condensing lens 51 and the pin hole plate 53; and a collimator lens 54. Incidentally, for the common light source 50, an LED (light-emitting diode) is used.

Infrared light emitted from the common light source 50 is focused with the condensing lens 51. The focused infrared light passes the aperture 52 and is led to the pin hole plate 53. A light flux passed through the pin hole plate 53 is caused to become a parallel light flux by the collimator lens 54, and is led to the cornea C of the subject's eye.

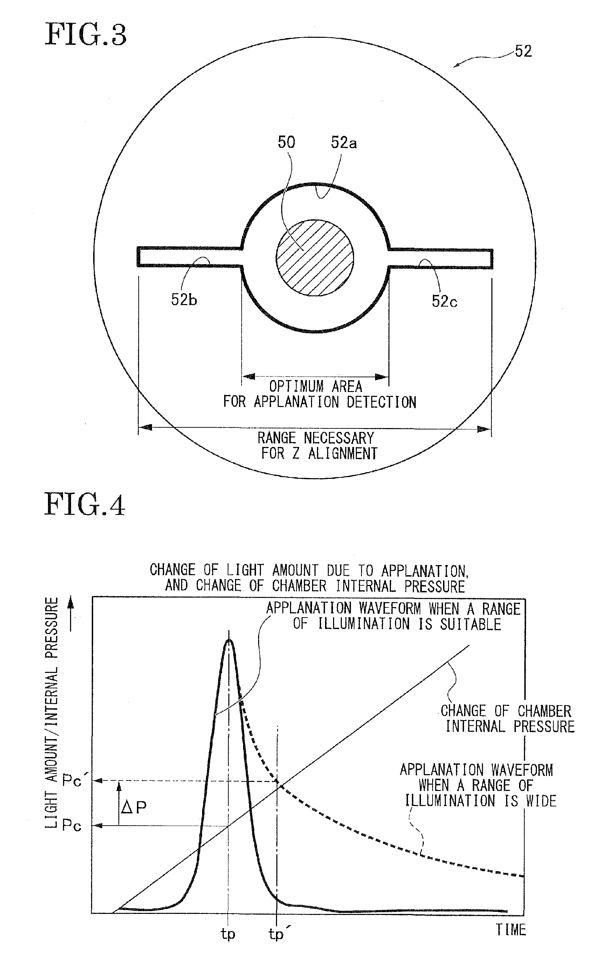

The above-described light flux shape regulator is, for example, an opening of the aperture 52, which will be described later. Further, the light flux shape regulator may regulate the projection light such that the projection light has a circular light flux portion (circular light flux), and slit-like light flux portions (slit-like light fluxes) extending in the lateral direction on both sides of the circular light flux portion, from the circular light flux portion. As shown in FIG. 3, the opening of the aperture 52 may include a circular opening portion 52a disposed in the middle of the aperture, and slit-like opening portions 52b, 52c formed on both sides of the circular opening portion 52a. A combination of the slit-like light flues led to the cornea C of the subject' s eye for detecting a Z alignment and the circular light flux for detecting applanation is reflected by the surface of the cornea. Incidentally, the aperture 52 is disposed on a position conjugate to a cornea vertex with respect to the collimator lens 54.

### [Z Alignment/Applanation Detection Light Receiving System]

The Z alignment/applanation detection light receiving system 6 is an optical system which includes, in a shared manner, a Z alignment light receiving system detecting a distance (working distance) between the instrument case 21 and the cornea C of the subject's eye, and an applanation detection light receiving system detecting a change in the shape of the cornea C of the subject's eye as a change in a light amount.

As shown in FIG.1, the Z aligmment/applanation detection light receiving system 6 includes: on a reflection optical axis 6L from the cornea C of the subject's eye, a condenser lens 60 and a half mirror 61; on an optical axis of a light flux reflected by the half mirror 61, an imaging lens 62 and a Z alignment light receiving sensor 63 detecting a light amount for moving in the direction of the Z axis; and on an optical axis of a light flux transmitting through the half mirror 61, an imaging lens 64 and an applanation detection light receiving sensor 65 detecting a light amount for detecting a change in shape of the subject's eye E. Incidentally, for the Z alignment light receiving sensor 63, a PSD sensor is used as in the XY alignment light receiving sensor 42.

A light flux reflected by the surface of the cornea C of the subject's eye is focused by the condensing lens 60, and the focused light flux is reflected by the half mirror 61 and, thereafter, caused to form a luminescent spot image on the Z alignment light receiving sensor 63 with the imaging lens 62 interposed therebetween. The light flux reflected by the surface of the cornea C of the subject's eye is focused by the condenser lens 60. The focused light flux transmits through the half mirror 61 and is, thereafter, caused to form, on the applanation detection light receiving sensor 65, the luminescent spot image in which a light amount changes depending on an amount of applanation of the cornea C of the subj ect' s eye. The imaging lens 64 is interposed between the half mirror 61 and the applanation detection light receiving sensor 65. Incidentally, the half mirror 61, for example, reflects the half of the light flux reflected by the surface of the cornea C of the subject's eye and transmits the remaining half of the light flux.

The above-described alignment portion measures a distance between the compressed air injection structure 1, e.g., the nozzle 11, and the subject's eye E based on the reflected light amount received by the Z alignment/applanation detection light receiving system 6. Then the compressed air injection structure 1 is positioned in the direction of the Z axis based on the measured distance. For example, the alignment portion moves the compressed air injection structure 1 in the direction of the Z axis along with the Z alignment/applanation detection projection system 5 and the Z alignment/applanation detection light receiving system 6.

The above-described alignment portion moves the Z alignment/applanation detection projection system 5 and the Z alignment/applanation detection light receiving system 6 in the direction of the Z axis and positions them such that a circular light flux projected from the common light source 50 is projected on the cornea of the subject`s eye E. Here, the non-contact type tonometer of the first embodiment includes an alignment auto-control circuit as the alignment portion. The alignment auto control circuit controls the drive of, for example, a motor which moves the instrument case 21 in the X, Y, and Z directions illustrated in the drawings based on detection signals from the XY alignment light receiving sensor 42 and the Z alignment light receiving sensor 63. And thereby, the positional relationships between instrument case 21 and the cornea C of the subject's eye in the X, Y, and Z directions is adjusted to a predetermined position (a position at which a light flux from the common light source 50 is reflected in the middle of the surface of the cornea C of the subject's eye). That is, the non-contact type tonometer of the first embodiment is an auto-alignment tonometer which automatically performs an alignment adjustment. Further, the alignment portion may include a joy-stick or the like for performing the alignment manually.

FIG.3 is an enlarged front view showing the aperture 52 of the Z alignment/applanation detection projection system 5 of the non-contact type tonometer of the first embodiment. A specific configuration of the aperture 52 is described below.

The light flux from the common light source 50 is projected to the pin hole plate 53 via the condenser lens 51 interposed therebetween. The light flux passed from the pin hole plate 53 is caused to become parallel light by the collimator lens 54 so as to illuminate the cornea C of the subject's eye. Between the condenser lens 51 and the pin hole plate 53, the aperture 52 which determines brightness of the optical system is disposed. The aperture 52 is disposed on a position substantially conjugate to the cornea C of the subj ect' s eye. Therefore, a range of illumination on the cornea C of the subject's eye is determined depending on the size and shape of the aperture 52.

Meanwhile, in the Z alignment projection system, when the subject's eye E moves in the Z direction (in forward and backward directions or in a longitudinal direction), an effective range of illumination on the cornea C of the subject's eye moves in the X direction (in left and right directions or in a lateral direction) but does not move in the Y direction (in upward and downward directions or in a vertical direction). Therefore, the range of illumination extending widely in the lateral direction is required. Further, in the case of the applanation detection projection system, the shape of illumination is preferably circular, and the area thereof is preferably close to a predetermined one. In order to satisfy both of these conditions, the shape of the light flux illuminating the cornea C of the subject' s eye is regulated (light flux shape regulated means) so as to have the circular light flux portion for illuminating the cornea and slit-like light flux portions, for detecting distance, which extends from the both sides of the circular light flux portion. At this time, it is preferable that a width of the projection light from the common light source 50 regulated so as to be the circular light flux portion is set to optimum size for detecting a deformation of the cornea of the subject's eye E and a width of the projection light from the common light source 50 regulated so as to be the slit-like light flux portions in the vertical direction is smaller than that of the circular light flux portion.

The shape of the light flux illuminating the cornea C of the subject's eye is regulated by the shape of the opening of the aperture 52. As shown in FIG. 3, the aperture 52 includes the circular opening portion 52a disposed in the middle of the aperture 52 and producing a circular light flux for cornea illumination, and the slit-like opening portions 52b, 52c disposed on the left and right sides of the circular opening portion 52a and producing slit-like light fluxes for distance detection. That is, the aperture 52 is one having a combination of a circular illumination range necessary for measuring applanation and a laterally long slit-like illumination range for covering a detection range of the Z alignment.

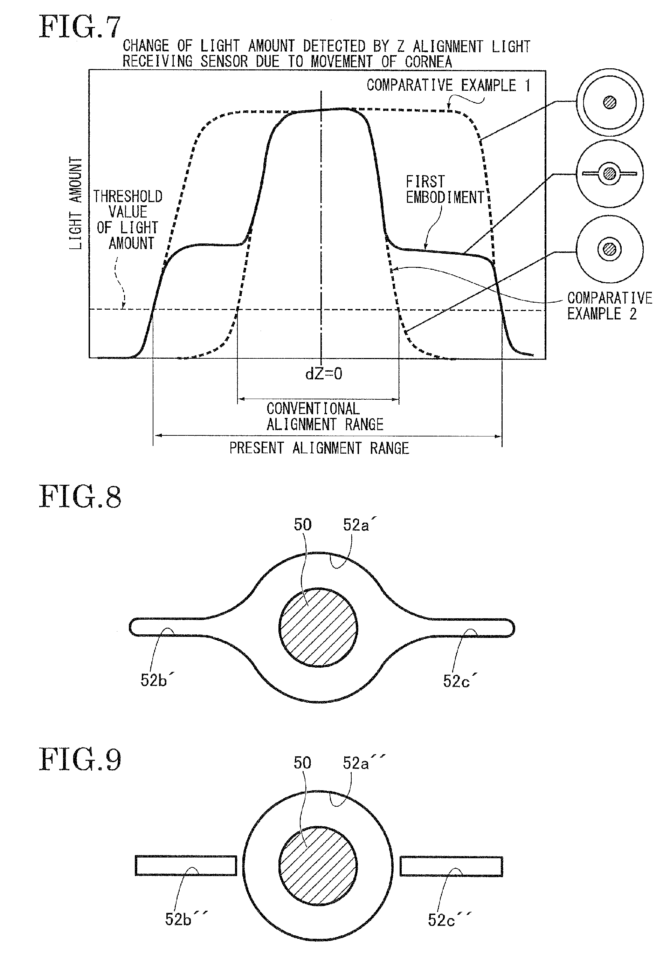

Here, the opening of the aperture 52 is made by an etching process. For example, the circular opening portion 52a has a diameter of 1.5 mm, and each of the slit-like opening portions 52b, 52c has a slit width of 0.2mm. In this manner, when the slit width of the slit-like opening portions 52b, 52c is smaller, an increase in the opening area can be controlled to a minimum, and a distortion of an applanation waveform is kept small, so that an influence of light fluxes from the slit like openings on a measurement can be controlled to a negligibly small range. On a range of the Z alignment, when an effective light flux moves from the circular opening portion 52a to the slit-like opening portions 52b, 52c, a light amount entering the Z alignment light receiving sensor 63 is reduced. But it is only necessary to secure light amount larger than a threshold amount which is an amount sufficient enough for acquiring a position (refer to FIG.7). Incidentally, as shown in FIG. 3, the common light source 50 is set in the middle of the circular opening portion 52a. In other words, a circular opening portion of the opening is disposed in a coaxial state with the Z alignment/applanation detection projection system 5.

Next, the technology of the non-contact type tonometer is described. The non-contact type tonometer blows air from the nozzle so as to applanate the cornea of a subj ect' s eye, and measures the intraocular pressure value of the subject's eye from the internal pressure of the chamber at the time when the cornea deforms into a certain shape.

The above-described intraocular pressure detection portion is capable of detecting a deformation of the subject's eye E based mainly on the light amount of the light flux projected on the subject's eye E as the circular light flux portion and reflected by the subject's eye E. Further, the above-described alignment portion is capable of moving the Z alignment/applanation detection projection system 5 and the Z alignment/applanation detection light receiving system 6 in the direction of the Z axis based on the light amount of the light fluxes projected on the subject's eye E as the slit-like light flux portions and reflected by the subject's eye E.

For this non-contact type tonometer, when adopting the common light source in the alignment projection system and the appl anation detection projection system in order to reduce optical projection systems and simplify the configuration of the optical systems, it is possible to consider a case where: (1) a fixed aperture structure using a circular shape; and (2) a variable aperture structure in which an aperture shape is switched are used for the illumination aperture.

### (1) The case where a fixed aperture structure using a circular shape is used.

In order to acquire an applanation waveform as a bilateral waveform, it is preferable to set the diameter of the light flux projected on the cornea of a subject's eye small. However, when the diameter of the light flux is set small, detection ranges of the XY alignment light receiving sensor and the Z alignment light receiving sensor become small. For this reason, there is a trade-off relationship between an extension of the illumination range for alignment and ensuring a measurement accuracy of the intraocular pressure value, and both can not be achieved at the same time.

### (2) The case where a variable aperture structure in which an aperture shape is switched is used.

When measuring an intraocular pressure using the non-contact type tonometer, the subject's eye is caused to view the fixation target in order to fix the subject' s eye in a predetermined position. However, in practice, since the subject's eye is constantly moving due to flicks or the like, the air must be blown to the cornea of the subject' s eye immediately after completing the alignment adjustment so as to make a measurement. Therefore, when the variable aperture structure in which the aperture shape is changed is used, after completing the alignment adjustment, there is an operation time until the variable aperture is stopped to a predetermined magnitude or the switching the aperture. While waiting for the operation to complete, the subj ect' s eye moves and the intraocular pressure cannot be measured.

Further, in the case of a tonometer in which observer manually makes an alignment adjustment while viewing an image on a monitor, a detection range does need to be so large. However, since the introduction of auto-alignment tonometers which automatically make an alignment adjustment, auto-alignment tonometers have be en spreading gradually. This is because troublesome manual alignment adjustment operation can be eliminated. In the case of such a tonometer, an alignment adjustment range needs to be set as large as possible, and it is preferable that the range of illumination is large.

Meanwhile, when the range of illumination for detecting applanation is unnecessarily large, the width of an applanation waveform is large and the waveform becomes asymmetric. This leads to an increase of error in a calculation of a center of gravity for obtaining an intraocular pressure value. That is, it is desirable that the range of illumination is kept as small as possible when detecting applanation.

For such a request, the inventors have focused on the point in that Z alignment is a one-dimensional coordinate system which is different from the XY alignment, being a two-dimensional coordinate system and an effective range of illumination on the cornea of the subject's eye moves only in the X direction but does not move in the Y direction when the subject' s eye moves in the Z direction.

In accordance with this point, in order to achieve both the securement of the accuracy of an intraocular pressure value by applanation and the securement of the Z alignment range while reducing optical projection systems, an aperture is adopted, which has a configuration made by combining a slit-like laterally long range of illumination, covering a detection range of the Z alignment, with a circular illumination field necessary for measuring an applanation.

Next, operations are described.

Described separately below are operations of the non-contact type tonometer of the first embodiment, i.e., an "intraocular pressure measurement operation," an "operation for securing a measurement accuracy of an intraocular pressure value," and an "operation for extending the range of a Z alignment adjustment".

### [Intraocular Pressure Measurement Operation]

A measurement of the intraocular pressure by the non-contact type tonometer of the first embodiment is as follows.

A subject fixes his/her face to supporters for a forehead and a jaw, and views the luminescent spot in the fixation target projection system with the subject' s eye. Under this state, the non-contact type tonometer automatically makes the alignment adjustment to the subject's eye E based on the sensor signals from the XY alignment light receiving sensor 42 of an XY alignment system and from the Z alignment light receiving sensor 63 of a Z alignment system.

Further, when a coincidence between all alignments in XYZ is confirmed, the solenoid is activated and the air from the nozzle 11 is blown to the cornea C of the subject's eye. At this time, the light amount entering the applanation detection light receiving sensor 65 of an applanation detection system, and a change in an internal pressure according to time detected by the chamber internal pressure sensor are stored.

The applanation detection system is configured such that when the cornea C of the subject's eye becomes flat by an applanation, the light amount entering the applanation detection light receiving sensor 65 becomes maximum. Thus, when the air is blown to the cornea C of the subject's eye, the light amount entering the applanation detection light receiving sensor 65 increases, in accordance with the applanation of the cornea C of the subject's eye, as shown by characteristics represented by a solid line of FIG.4, and becomes maximum at time tp when the cornea C of the subj ect' s eye becomes flat. When the applanation progresses further and the cornea C of the subject's eye becomes concave, the light amount received by the applanation detection light receiving sensor 65 decreases.

The position (a position at the time tp) of the center of gravity of the light amount changing with time is detected, and the chamber internal pressure value Pc at this time is obtained. Here, since there is a certain relationship between the internal pressure of the chamber 10 and the pressure of the air blown to the cornea C of the subject's eye, the intraocular pressure value of the subject's eye can be obtained from the chamber internal pressure value Pc.

### [Operation for securing a measurement accuracy of an intraocular pressure value]

As described above, the applanate detection system is configured such that the light amount entering the applanate detection light receiving sensor 65 changes (from an increased state to a decreased state via a maximum state) in accordance with the applanation of the cornea C of the subject's eye.

For example, assume a case where a circular opening portion 52a in the middle of the aperture 52 is set to have a shape such that the area thereof is larger than the area optimum for the applanate detection shown in FIG. 3. In this case, the light amount of the light entering the applanation detection light receiving sensor 65 increases in accordance with the applanation of the cornea C of the subject's eye, and becomes maximum at the time tp when the cornea C of the subject's eye becomes flat (characteristic which coincides with the characteristics represented by the solid line of FIG.4). When the applanation progresses further and the cornea C of the subject's eye becomes concave, the light amount of the light entering the applanation detection light receiving sensor 65 decreases. However, the applanation detection light receiving sensor 65 also receives reflected light from a peripheral part where the cornea is deformed. This is caused because of an increase in the diameter of the circular opening portion. As shown by the characteristics represented by the dotted line of FIG.4, the light amount of the light entering the applanation detection light receiving sensor 65 is increased and a slope indicating the decrease in the light amount becomes moderate.

Therefore, when the position (a position at a time tp') of the center of gravity of the light amount changing with time in characteristics represented by a dotted line of FIG. 4 is detected, and a chamber internal pressure value Pc' is obtained at this time, the chamber internal pressure value Pc' becomes larger than the chamber internal pressure Pc by ΔP. Thus, there occurs an error in an intraocular pressure value of the subject's eye obtained based on the chamber internal pressure.

In contrast, in the first embodiment, the shape of the circular opening portion 52a in the middle of the aperture 52 is set to have an area which is optimum for an applanation detection as shown in FIG. 3. Thus, as shown by the characteristics represented by the solid line of FIG. 4, an applanation waveform having a single sharp peak, which is obtained when the illumination range is suitable, is obtained. Accordingly, the position (a position at time tp) of the center of gravity of the light amount changing with time approximately coincides with the peak position of the light amount, so that a high measurement accuracy of an intraocular pressure value can be secured.

### [Operation of Extending the Range of a Z Alignment Adjustment]

A working distance which is a distance between the cornea C of the subject's eye and the instrument can be obtained as described below, using the Z alignment system.

The light flux from the common light source 50 is caused to be a parallel light flux by the collimator lens 54, and the parallel light flux obliquely illuminates the subject's eye E. The light flux reflected by the cornea C of the subject's eye reaches the Z alignment light receiving sensor 63 via the lens system. The light flux enters the cornea C of the subject's eye as parallel light flux and is reflected by the cornea C of the subject's eye, and is caused to form a virtual image, namely a Purkinj e image, on a position which is in the middle (r/2) of the radius of curvature of the cornea C of the subject's eye. The "Purkinje image" is a luminescent spot image which is occurs by reflecting, with the cornea serving as a mirror, a light source outside the eyeball, and the light source seems to be inside the eyeball.

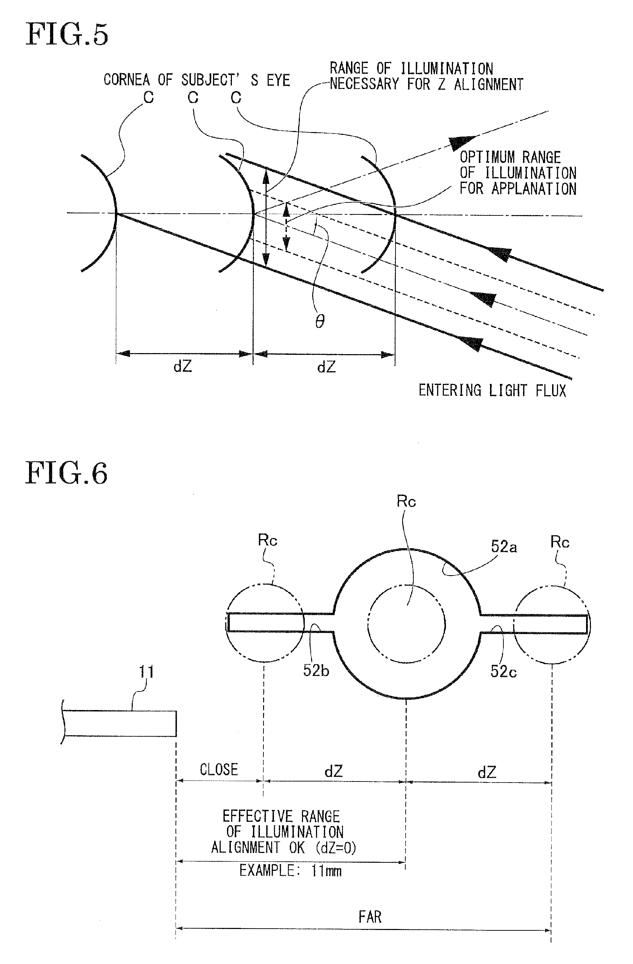

The Z alignment light receiving sensor 63 of the Z alignment light receiving system is disposed on a position which is substantially conjugate to the Purkinje image. Therefore, as shown in FIG.5, since a light flux entering the cornea C of the subject's eye has an angle θ, when the distance between the cornea C of the subject's eye and the instrument changes from an alignment OK position, a luminescent spot on the Z alignment light receiving sensor 63 moves in the lateral direction. That is, when the distance between the cornea C of the subject's eye and the instrument (e.g., the nozzle 11) is changed to come closer to each other by a distance dZ from the alignment OK position (a position where dZ is 0: for example, the position which is 11 mm away from the nozzle 11), a reflected light flux Rc from the cornea of the subject's eye in a deformed state laterally moves, on the Z alignment light receiving sensor 63, from the position within the circular opening portion 52a to the slit-like opening portion 52b. On the other hand, when the distance between the cornea C of the subject's eye and the instrument (e.g., the nozzle 11) is changed to a longer distance, that is, the cornea C of the subject's eye is moved from the alignment OK position by a distance dz, the reflected light flux Rc from the cornea C of a subject's eye in a deformed state laterally moves, on the Z alignment light receiving sensor 63, from the position within the circular opening portion 52a to the slit-like opening portion 52c.

Therefore, from a movement amount of the luminescent spot, a moving distance (working distance) of the cornea of the subject's eye from a reference position in an axis direction (a direction in which the air is blown, that is, the direction of the reference axis) can be detected. Incidentally, the movement of the luminescent spot occurs when the subject's eye moves within a plane (within an XY plane) perpendicular to the air blowing axis. Thus, the working distance is corrected using information obtained from the XY alignment system, or the working distance is obtained after the XY alignment adjustment is completed so that there are no out of alignment in the XY plane.

FIG.7 is a view showing comparative characteristics showing changes in a light amount received by the Z alignment light receiving sensor 63 due to a movement of the cornea C of the subject's eye. The characteristics of change of the light amount received by the applanation detection light receiving sensor 65 are also the same.

As shown in FIG.7, the light flux shape regulator regulates the projection light from the common light source 60 to have first projection light fluxes on both ends in the lateral direction and a second projection light flux in the middle of the first projection light fluxes. Here, it is preferable that an amount of each of first reflected light fluxes projected as the first projection light fluxes and reflected at the subject's eye E be equal to be or larger than a threshold value at which the Z alignment light receiving sensor can receive light, that is, is in a light-detectable state. And also, it is preferable that an amount of a second reflected light flux projected as the second projection light flux and reflected by the subj ect' s eye E to be larger than the amount of the first reflected light fluxes.

First, Comparative Example 1 is defined by an example which has, as the opening for the aperture, a large circular opening portion that regulates the circular light flux having a large diameter for cornea illumination and distance detection, and in which the light source is disposed in the middle of this large circular opening portion. Here, the securing of a Z alignment range is given priority. In the case of this Comparative Example 1, as shown by characteristics represented by a dotted curve of FIG.7, a light amount greatly exceeds a threshold value within a wide range of movement amount, the range extending in the forward and backward directions of a Z alignment normal position (dZ=0). The characteristic changes in light amount form a trapezoid shape. Thus, an extension of the Z alignment range is achieved. However, an amount of leakage light increases when measuring an intraocular pressure and, consequently, an applanation waveform having a single sharp peak cannot be acquired, so that a measurement accuracy of an intraocular pressure is deteriorated.

Next, Comparative Example 2 is defined by an example which has, as the opening for the aperture, a circular opening portion that regulates a circular light flux for cornea illumination and distance detection, and in which a light source is disposed in the middle of this circular opening portion. Here an applanation measurement is given priority. In the case of this Comparative Example 2, as shown by characteristics represented by a broken curve of FIG.7, a light amount greatly exceeds the threshold value within a small range of movement amount, the range extending in the forward and backward directions of a Z alignment normal position (dZ=0). The characteristic changes in light amount form a trapezoid shape. Thus, an applanation waveform having a single sharp peak, acquired when the range of illumination is suitable, can be acquired at the time of measurement of an intraocular pressure, so that a high measurement accuracy of an intraocular pressure can be achieved. However, the Z alignment range becomes a narrow range (equal to a conventional alignment range) between two points at which the broken characteristic curve and a line representing the light amount threshold value intersect in FIG.7, so that this is not suitable for an auto-alignment tonometer.

In contrast, similarly to the case in the first embodiment, the opening of the aperture 52 includes, as shown in FIG.3, the circular opening portion 52a, in the middle thereof, creating the circular light flux for cornea illumination and the slit-like opening portions 52b, 52c on left and right positions creating the slit-like fluxes of light for distance detection. Further, the common light source 50 is set in the middle of the circular opening portion 52a.

Therefore, as shown in characteristics by a solid curve of FIG.7, the characteristics are represented by the combination of two trapezoidal characteristics. One trapezoidal characteristic portion shows a light amount, which greatly exceeds the light amount threshold value within a small range of movement amount, the range extending in the forward and backward directions of a Z alignment normal position (dZ=0). And another trapezoidal characteristic portion shows a light amount, which slightly exceeds the threshold value within a wide range of movement amount, the range extending in the forward and backward direction of a Z alignment normal position (dZ=0).

Accordingly, an applanation waveform having a single sharp peak, which can be acquired when the range of illumination is suitable, can be acquired at the time of measurement of an intraocular pressure. Thus, a high measurement accuracy of an intraocular pressure can be secured. In particular, since the slits of the slit-like opening portions 52b, 52c are set to have a small width, an increase in the opening area compared to the opening area of the circular opening portion 52a can be controlled to a minimum and a distortion of the applanation waveform is also small, so that an influence on a measurement can be controlled to a negligibly small range.

In addition, the Z alignment range becomes a wide one (=the present alignment range) between two points at which the solid characteristic curve and a line representing the light amount threshold value in FIG.7 intersect. Thus, this wide Z alignment range is suitable for an auto-alignment tonometer automatically making an alignment adjustment. For this Z alignment range, when an effective light flux moves from the circular opening portion 52a to the slit-like opening portions 52b, 52c, a light amount of the light entering the Z alignment light receiving sensor 63 decreases, but a range, in which a light amount larger than a threshold value sufficient for obtaining a position is secured, can be used as an adjustment range for the Z alignment.

Next, effects are described.

The non-contact type tonometer of the first embodiment can provide the following effects.
(1) The non-contact type tonometer which blows air to the cornea C of the subject's eye, detects a deformed state of the cornea C of the subject's eye as a change of a reflected light amount, and measures an intraocular pressure value of the subject's eye, includes: the common light source 50 which is commonly used as a light source for illuminating the cornea C of the subject's eye for measuring the intraocular pressure value and for illuminating the subject's eye E to detect a distance between the instrument and the subject's eye E; and the light flux shape regulator which is set on a light path from the common light source 50 to the subject's eye E and which regulates a light flux, illuminating the cornea C of the subject's eye, to one having a laterally long non-circular shape. Therefore, the non-contact type tonometer of the first embodiment can achieve both the securement of a measurement accuracy of an intraocular pressure value by applanation and the securement of the Z alignment range, while making the optical system compact and comparatively simple.
(2) The light flux shape regulator regulates the shape of the light flux, illuminating the cornea C of the subject's eye, to have the circular light flux portion for cornea illumination and slit-like light flux portions, for distance detection, extending from the circular light flux portion to both sides. The light flux shape regulator is capable of securing high measurement accuracy of an intraocular pressure value by the applanation waveform having a peak, and at the same time extending the alignment adjustment range without deteriorating the measurement accuracy of an intraocular pressure value.
(3) Provided is the Z alignment/applanation detection projection system 5 in which between the common light source 50 and the cornea C of the subject's eye, the condensing lens 51, the illumination aperture 52, the pin hole plate 53, and the collimator lens 54 are disposed, and a light flux passing the pin hole plate 53 is caused to be a parallel light flux with the collimator lens 54 so as to illuminate the cornea C of the subject's eye. The light flux shape regulator is disposed between the condensing lens 51 and the pin hole plate 53, and regulates the light flux by the shape of the opening of the aperture 52 disposed on a position substantially conjugate to the cornea C of the subject's eye. Therefore, by using a simple method based on a determination of the shape of the opening of the aperture 52, the shape of the light flux illuminating the cornea C of the subject's eye can be regulated to a desired one.
(4) The opening of the aperture 52 includes: the circular opening portion 52a, in the middle thereof, creating the circular light flux for cornea illumination; and slit-like opening portions 52b, 52c, on the left and right positions of the circular opening portion 52a, creating the slit-like fluxes of light for distance detection. The common light source 50 is set in the middle of the circular opening portion 52a of the aperture 52. Therefore, a measurement accuracy of an intraocular pressure value can be improved by a single applanation waveform having a sharp peak, and at the same time an adjustment range of the Z alignment can be greatly extended while controlling a distortion of an applanation waveform to a minimum.
(5) Provided is the Z alignment/applanation detection light receiving system 6 including the Z alignment light receiving sensor 63 and the applanation detection light receiving sensor 65 both of which receive via the lens system the light flux entering the cornea C of the subject's eye in an oblique direction with an angle θ and reflected by the cornea C of the subject's eye. Here, in terms of change characteristics of a movement amount of the cornea C of the subject's eye to the instrument, and a light amount received by the Z alignment/applanation detection light receiving system 6, the light amount threshold value is defined as a minimal light amount needed for detection of Z alignment. By use of the defined light amount threshold value, the light flux shape regulator regulates the shape of a light flux illuminating the cornea C of the subject's eye to obtain combined characteristics of two trapezoidal characteristic portions. One of the trapezoidal characteristic portions is a portion indicating a light amount which greatly exceeds the light amount threshold value within a small range of movement amount, the range extending in the forward and backward directions of the Z alignment normal position. The other trapezoidal characteristic portion is a portion indicating a light amount which slightly exceeds the light amount threshold value within a wide range of movement amount, the range extending in the forward and backward directions of the Z alignment normal position. Therefore, an intraocular pressure value can be measured with high accuracy based on an applanation waveform due to a suitable change of detected light amount, and at the same time the Z alignment adjustment range can be extended due to a sufficient amount of detected light for obtaining the position of the Z alignment.
(6) Since the Z alignment light receiving sensor 63 is the PSD sensor using a semiconductor position detection element for detecting the position of a spot-shaped light, the Z alignment light receiving sensor 63 can accurately detect the Z alignment position even if there is a small change in a detected light amount.

The non-contact type tonometer of this invention has been described based on the first embodiment, but the specific configuration is not limited to that of the first embodiment. Changes in design, additions, and the like may be made within the scope of the present invention, which does not depart from the points of present invention.

In the first embodiment, as the light flux shape regulator, the example has been shown in which a light flux is regulated by the shape of the opening of the aperture 52, but in the Z alignment/applanation detection projection system, for example, it is possible to determine only the brightness of the optical system by the aperture and separately provide the light flux shape regulator regulating the shape of a parallel light made by the collimator lens.

In the first embodiment as the opening of the aperture 52, the example has been shown, which includes the circular opening portion 52a, in the middle thereof, creating the circular light flux for cornea illumination, and the slit-like opening portions 52b, 52c, on the left and right positions of the circular opening portion 52a, creating the slit-like fluxes of light for distance detection. However, for example, it is possible to consider a variation, as shown in FIG.8, in which a circular opening portion 52a' and slit-like opening portions 52b', 52c' are connected by gentle curves, or another variation, as shown in FIG.9, in which a circular opening portion 52a" and slit-like opening portions 52b", 52c" are separately provided via narrow connecting portion. In short, the specific shape is not necessarily limited to that in the first embodiment as long as the shape presents a light amount change characteristic of the light receiving sensor is such as the solid curve characteristic of FIG.7.

Therefore, in the non-contact type tonometer according to one embodiment of the present invention, a common light source simultaneously serves as a light source (a light source for applanation detection) illuminating a cornea of a subject's eye for measuring an intraocular pressure value and as a light source (a light source for Z alignment) illuminating the subject's eye for detecting a distance (=Z alignment) between a instrument and the subject's eye, so that the number of optical systems each having a light source can be reduced. In addition, by the light flux shape regulator set on a light path from the common light source to the subject's eye, the shape of a light flux illuminating the cornea of the subject's eye is regulated to a laterally long non-circular shape. Thus, for example, when the shape is formed by combining laterally long slit-like portions and a circular portion, the measurement accuracy of the intraocular pressure value due to an applanation can be secured by limiting a range of illumination to a small range using a light flux from the circular shape, and the Z alignment range can be secured by securing a wide range of illumination in the lateral direction using light fluxes from the slit-like portions. That is, in the Z alignment projection system and light receiving system, when the cornea of the subject's eye moves in the forward and backward directions (in the Z direction), an effective range of illumination on the cornea of the subject's eye moves in the left and right directions (in the X direction) but does not move in the upward and downward directions (in the Y direction). In other words, the extending of the range of illumination only in the X direction (in the lateral direction) causes an adjustment range of Z alignment to be extended. Consequently, both the securement of the measurement accuracy of an intraocular pressure value and the securement of the Z alignment range can be achieved while making the optical systems compact and relatively simple.

In the first embodiment, the example has been shown, in which the non-contact type tonometer automatically makes all the XYZ alignment adjustments, but the present invention is applicable to a non-contact type tonometer which automatically makes one of the XY alignment adjustments and the Z alignment adjustment, or to a non-contact type tonometer by which all the XYZ alignment adjustments are manually made. This means that the present invention can be applied to any non-contact type tonometer which blows air to a cornea of a subject's eye, detects a deformed state of the cornea of the subject's eye as changing reflected light amount, and measures the intraocular pressure value of the cornea of the subject' s eye.

## Claims

1. A non-contact type tonometer which deforms a cornea (C) of a subject's eye (E) in a non-contact state and measures an intraocular pressure value, comprising:
a compressed air injector (1) configured to blow air to the cornea of the subject's eye along a reference axis (Z) to deform the cornea of the subject's eye;
projection optical system (5) configured to project projection light onto the subject's eye;
a light receiving optical system (6) configured to receive reflected light which is projected from the projection optical system and reflected on the subject's eye;
an alignment unit configured to position the projection optical system and the light receiving optical system by moving the projection optical system and the light receiving optical system relative to the subject's eye in the direction of the reference axis, based on a light amount of the reflected light, which is received by the light receiving optical system; and
an intraocular pressure detector configured to detect a deformation of the cornea of the subject's eye deformed by the compressed air injector, based on the reflected light amount received by the light receiving optical system,
wherein the projection optical system includes a light flux shape regulator (52) which regulates the projection light such that the projection light has a non-circular shape extending in a predetermined direction.

2. The non-contact type tonometer according to claim 1, wherein the alignment unit is configured to measure a distance between the compressed air injector and the subject's eye based on the reflected light amount received by the light receiving optical system.

3. The non-contact type tonometer according to claim 2, wherein the alignment unit is configured to position the compressed air injector in the direction of the reference axis, based on the measured distance.

4. The non-contact type tonometer according to claim 1, wherein the alignment unit is configured to move the compressed air injector in the direction of the reference axis together with the projection optical system and the light receiving optical system.

5. The non-contact type tonometer according to claim 1, wherein the light flux shape regulator is configured to regulate the projection light such that the projection light has a circular light flux portion and slit-like light flux portions extending in the predetermined direction from the circular light flux portion on both sides of the circular light flux portion.

6. The non-contact type tonometer according to claim 1, wherein the intraocular pressure detector is configured to detect an intraocular pressure of the subject's eye based on a change of the light amount received by the light receiving optical system.

7. The non-contact type tonometer according to claim 1, wherein the projection optical system includes a single light source (50).

8. The non-contact type tonometer according to claim 5, wherein
the intraocular pressure detector is configured to detect the deformation of the subject's eye based on an amount of a reflected light flux which is projected on the subj ect' s eye as the circular light flux portion and is reflected on the subject's eye; and
the alignment unit is configured to move the projection optical system and the light receiving optical system in the direction of the reference axis based on an amount of each of light fluxes which are projected on the subject's eye as the slit-like light flux portions and are reflected on the subject's eye.

9. The non-contact type tonometer according to claim 1, wherein
the projection optical system includes a condensing lens (51), a pin hole plate (53), an aperture (52) and a collimator lens (54), the aperture being disposed on a position which is substantially conjugate to the subject's eye and between the condensing lens and the pin hole plate;
the projection optical system is configured to project the projection light on the subject's eye as parallel light; and
the light flux shape regulator is an opening of the aperture.

10. The non-contact type tonometer according to claim 9, wherein the opening of the aperture includes a circular opening portion (52a) disposed in a middle of the aperture, and slit-like opening portions (52b) formed on both sides of the circular opening portion.

11. The non-contact type tonometer according to claim 10, wherein
the projection optical system includes a single light source (50); and
the circular opening portion of the opening is disposed in a coaxial state with the projection optical system.

12. The non-contact type tonometer according to claim 1, wherein projection light is projected from the projection optical system with an angle in relation to the reference axis.

13. The non-contact type tonometer according to claim 1, wherein
the light receiving optical system includes a Z alignment light receiving sensor (63) which is configured to detect a light amount to move the projection optical system and the light receiving optical system in the direction of the reference axis, and an applanation detection light receiving sensor (65) which is configured to detect a light amount to detect the deformation of the subject's eye;
the light flux shape regulator is configured to regulate the projection light such that the projection light has first projection light fluxes on both ends of the projection light extending in the predetermined direction and a second projection light flux in the middle the projection light;
an amount of each of first reflected light fluxes which are projected as the first projection light fluxes and reflected by the subject's eye, is the same or more than a detectable threshold value of the Z alignment light receiving sensor; and
an amount of a second reflected light flux which is proj ected as the second projection light flux and reflected on the subject's eye, is larger than the amount of each of the first reflected light fluxes.

14. The non-contact type tonometer according to claim 13, wherein the Z alignment light receiving sensor is a PSD sensor using a semiconductor position detection element which is configured to detect a position of a spot of light.
